(19) Europäisches Patentamt
European Patent Office
Office européen des brevets

(11) Veröffentlichungsnummer: **0 259 752 A2**

(12) **EUROPÄISCHE PATENTANMELDUNG**

(21) Anmeldenummer: **87112718.9**

(51) Int. Cl.4: **C07H 11/04** , A61K 31/70

(22) Anmeldetag: **01.09.87**

(30) Priorität: **12.09.86 DE 3631004**

(43) Veröffentlichungstag der Anmeldung: **16.03.88 Patentblatt 88/11**

(84) Benannte Vertragsstaaten: **AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Hayauchi, Yutaka, Dr.**
**Gustav-Freytag-Strasse 4**
**D-5090 Leverkusen 1(DE)**
Erfinder: **Lockhoff, Oswald, Dr.**
**Morgengraben 14**
**D-5000 Köln 80(DE)**
Erfinder: **Babczinski, Peter, Dr.**
**In der Lohrenbeck 11**
**D-5600 Wuppertal 1(DE)**
Erfinder: **Petzinna, Dieter, Dr. Dr.**
**Peter-Berten-Strasse 10**
**D-4000 Duesseldorf 12(DE)**
Erfinder: **Bischoff, Hilmar, Dr.**
**Am Rohm 78**
**D-5600 Wuppertal 1(DE)**

(54) **Sacchharid-Phosphate und ihre Ester und Verfahren zu ihrer Herstellung.**

(57) Die Erfindung betrifft neue Saccharidphosphate der allgemeinen Formeln (I) und (II)

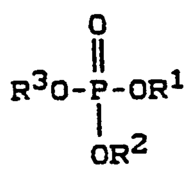

(I)

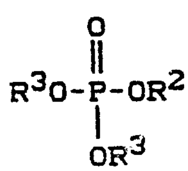

(II)

in welchen $R^1$, $R^2$ und $R^3$ die in der Beschreibung angegebene Bedeutung haben, ihre Ester und Verfahren zu ihrer Herstellung.

EP 0 259 752 A2

## Saccharid-Phosphate und ihre Ester und Verfahren zu ihrer Herstellung

Die Erfindung betrifft neue Saccharidphosphate und ihre Ester und Verfahren zu ihrer Herstellung. Die neuen Verbindungen entsprechen der allgemeinen Formel (I).

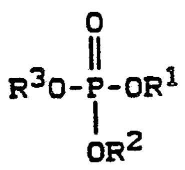

(I)

Hierin bedeutet $R^1$ einen gegebenenfalls substituierten geradkettigen oder verzweigten gesättigten oder einfach oder mehrfach ungesättigten Alkylrest, einen gegebenenfalls substituierten, gesättigten oder einen ein-oder mehrfach ungesättigten Cycloalkylrest, $R^2$ bedeutet Wasserstoff, Phosphatschutzgruppen, anorganisches oder organisches Kation,

$R^3$ bedeutet einen freien oder geschützten Saccharidrest der Pyranose-oder Furanoseform aus der Reihe der Hexosen oder Pentosen.

Der Rest $R^1$ in der Bedeutung Alkyl hat bis zu 40, vorzugsweise bis zu 20 C-Atome.

Beispiele für gesättigte Alkylreste $R^1$ sind Methyl, Ethyl, Propyl, Methylethyl, Butyl, Methylbutyl, Dimethylethyl, Pentyl, Methylbutyl, Ethylpropyl, Dimethylpropyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, Docosyl, Tetracosyl, Triacontyl,Ethylpentyl, Methyldecyl, Propyldecyl, Methyltridecyl, Pentylhexadecyl, Dodecylhexadecyl, Octadecyleicosyl, Hexadecyloctadecyl.

Ungesättigte Alkylreste $R^1$ sind beispielsweise Ethenyl, 1-Propenyl, 2-Propenyl, Butenyl, Methylpropenyl, Methylbutenyl, Pentenyl, Hexenyl, Octenyl, Nonenyl, Decenyl, 3,7-Dimethyl-2,6-octadienyl, Octadecenyl, Octadecadien-9,12-yl, Octadecatrien-9,12,15-yl, 3,7,11-Trimethyl-2,6,10-dodecatrien-1-yl, Butadienyl, Pentadienyl, Ethinyl, Propinyl, Butinyl, Pentinyl, Hexinyl, Heptinyl, Octinyl, Noninyl, Decinyl, Dodecinyl, Tetradecinyl, Hexadecinyl, Octadecinyl.

Die ungesättigten Kohlenwasserstoffreste können dabei als reine cis-oder trans-Isomere oder auch als Isomerengemische vorliegen.

$R^1$ in der Bedeutung Cycloalkyl steht bevorzugt für Reste mit 3 - 7 C-Atomen.

Beispiele für Cycloalkylreste sind Cyclopropyl, Cyclobutyl, Cyclopentyl, Cyclohexyl, Cycloheptyl, Cyclohexylcyclohexyl, Cyclopentylcycloheptyl.

Beispiele für ungesättigte cyclische Reste $R^1$ sind Cyclopentenyl, Cyclohexenyl und Cycloheptenyl.

Solche Cycloalkylringe können mit Alkylresten (bevorzugt 1 - 8 C) substituiert sein oder in eine Alkylkette mit bis zu 40 C-Atomen eingefügt sein. Beispielhaft hierfür seien genannt:

Methylcyclopentyl, Ethylcyclopentyl, n-Propylcyclopentyl, i-Propylcyclopentyl, Butylcyclopentyl, Octylcyclopentyl, Methylcyclohexyl, Ethylcyclohexyl, Propylcyclohexyl, Butylcyclohexyl, Hexylcyclohexyl, Decyl cyclohexyl, Cyclopentylmethyl, Cyclopentylethyl, Cyclopentylpropyl, Cyclopentylbutyl, Cyclopentylpentyl, Cyclopentylhexyl, Cyclopentyloctyl, Cyclopentyldecyl, Cyclohexylmethyl, Cyclohexylethyl, Cyclohexylpropyl, Cyclohexylbutyl, Cyclohexyldecyl, Cyclopentylcyclohexylethyl, Cyclohexylcyclopentylethyl und Cyclohexylcyclohexylethyl.

Die Substituenten können jeweils cis-oder transständig angeordnet sein.

Die Reste $R^1$ können substituiert sein, vorzugsweise durch Halogen, vorzugsweise F, Cl oder Br, Azid, Cyano oder Trailkylsilyl.

Beispiele für halogenierte Kohlenwasserstoffreste $R^1$ sind Fluorethyl, Chlorethyl, Bromethyl, Iodethyl, Trifluorethyl, Trichlorethyl, Tribromethyl, Fluorethenyl, Chlorethenyl, Bromethenyl, Iodethenyl, Fluorpropyl, Chlorpropyl, Brompropyl, Iodpropyl, Chlorbutyl, Brombutyl, Chlorpentyl, Brompentyl, Chlorhexyl, Bromhexyl, Chlorheptyl, Bromheptyl, Chloroctyl, Bromoctyl, Chlornonyl, Bromnonyl, Chlordecyl, Bromdecyl.

Der Rest $R^2$ bedeutet Phosphatschutzgruppen, Wasserstoff oder anorganisches oder organisches Kation.

Verwendung der Phosphatschutzfunktion ermöglicht nach einer nachgeschalteten Deblockierungsreaktion zur Phosphorsäurediestern mit $R^2$ = H oder Kation zu gelangen. Solche Schutzgruppen sind beispielsweise in der Nucleotidchemie geläufig (C.B. Reese, Tetrahedron 34, (1978) 3143-3179). Beispielhaft hierfür seien genannt: Aryl wie Phenyl, o-Chlorphenyl, p-Chlorphenyl, 2,4-Dinitrophenyl, p-Nitrophenyl, Aralkyl wie Benzyl, Benzhydryl, p-Methoxybenzyl, Triphenylmethyl, Haloalkyl wie 2,2,2-Trichlorethyl, 2-Cyanoethyl.

Wenn $R^2$ Wasserstoff ist, bilden die vorstehend beschriebenen Verbindungen saure Phosphorsäureester, die mit anorganischen oder organischen Basen Salze geben. Entsprechende anorganische Kationen sind beispielsweise Metallkationen wie $Li^+$, $Na^+$, $K^+$, $Mg^{2+}$, $Ca^{2+}$ oder $NH_4^+$. Als organisches Kation kommt ein geeignetes Ammoniumion zur Anwendung. Beispielhaft seinen genannt: Triethylammonium, Pyridinium, Tetraethylammonium, Tetrabutylammonium, Cyclohexylammonium, Dicyclohexylammonium.

$R^3$ bedeutet einen freien oder geschützten Saccharidrest der Pyranose-oder Furanoseform aus der Reihe der Hexosen oder Pentosen.

Beispiele für solche Zucker sind Ribose, Arabinose, Xylose, Lyxose, Allose, Altrose, Glucose, Mannose, Gulose, Idose, Galactose, Talose, Glucosamin, Galactosamin oder Mannosamin, sowohl in der D-Reihe als auch in der L-Reihe.

Die nicht phosphorylierten Hydroxylgruppen liegen entweder frei oder blockiert durch die vorstehend erläuterten Schutzgruppen vor.

Der Phosphorsäurerest kann esterartig an eine beliebige Hydroxygruppe der Saccharideinheit eingeführt sein. Bevorzugte Positionen der Phosphatestergruppen sind 6-OH der Hexopyranosen und -furanosen, 5-OH der Pentofuranosen sowie α-oder β-anomere Hydroxygruppe der Hexosen und Pentosen. Weitere bevorzugte Saccharidkomponenten $R^3$-OH sind 1,2,:3,4-Di-O-isopropyliden-α-D-galactopyranose, 2,3:5,6-Di-O-isopropyliden-α-D-mannofuranose, 1,2:5,6-Di-O-isopropyliden-α-D-glucofuranose, 1,2,3,4-Tetra-O-acetyl-D-gluco-, -galacto-oder -mannopyranose, 2-Acetamido-1,3,4-tri-O-acetyl-2-desoxy-D-glucopyranose, 2,3,4,6-Tetra-O-benzyl-D-gluco-, -galacto-oder -mannopyranose, 2,3,4,6-Tetra-O-acetyl-D-gluco-, -galacto-oder -mannopyranose, 2-Acetamido-3,4,6-tri-O-acetyl-2-desoxy-D-glucopyranose, Benzyl 2,3-O-isopropyliden-β-D-ribofuranosid.

Die Verbindungen der Formel (I) enthalten mehrere chirale C-Atome und liegen als optisch reine Diastereomere oder als Diastereomerengemische vor.

Die Erfindung betrifft auch Verfahren zur Herstellung der Verbindung gemäß Formel (I)

Phosphortriester

Bei der Herstellung der Verbindungen (I) setzt man die von Formel (I) umfaßten Zuckerkomponenten (V) entweder in ungeschützter Form oder in Form geeigneterweise ge schützter Derivate um mit einem Phosphorochloridat (IV) gemäß Formel schema (III)

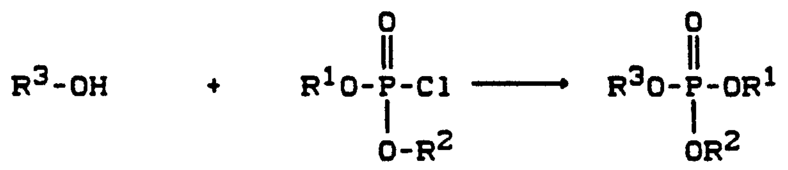

(III)

(V) (IV) (I)

wobei $R^1$, $R^2$ und $R^3$ wie vorstehend beschrieben sind.

Phosphorochloridat (IV) in der Reaktionsgleichung (III) wird wiederum gemäß dem folgenden Schema (VI) in vorgelagertem Schritt synthetisiert (H. Eibl. Angew. Chem., 26 (1984) 247-314).

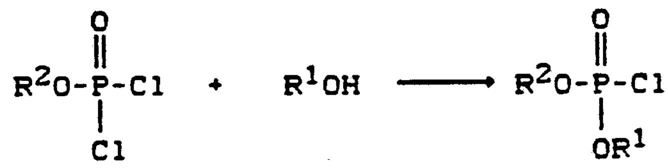 (VI)

(VII) (VIII) (IV)

wobei $R^1$ und $R^2$ wie vorstehend beschrieben sind.

Bei einer bevorzugten Ausführung der Reaktion (VI) wird das Phosphorodichloridat (VII) in einem Lösungsmittel vorgelegt und eine Lösung des Alkohols $R^1OH$ und einer Base in demselben Lösungsmittel langsam zugetropft. Als Lösungsmittel kommt ein geeignetes aprotisches Lösungsmittel zur Verwendung wie Dimethylformamid, Aceton, Ethylacetat, Tetrahydrofuran, Dioxan, Diethylether, Chloroform, Dichlormethan, Dichlorethan, Benzol, Toluol, Petrolether, Heptan, Hexan, Pentan, Bevorzugt sind Petrolether, Heptan, Hexan, Dichlormethan, Dichlorethan, Chloroform sowie ein Mischlösungsmittel aus diesen. Die Reaktionstemperatur liegt zwischen -40°C und 100°C, vorzugsweise bei -20°C bis zur Raumtemperatur. Die Umsetzung dauert von einer halben Stunde bis 24 Stunden, vorzugsweise bis zu drei Stunden.

Als Base kann man ein tertiäres Amin, metallierte Alkyle und Amine oder Metallhydride verwenden wie Pyridin, Triethylamin, Diisopropylethylamin, 2,4,6-Trimethylpyridin, Butyllithium, Lithiumdiisopropylamin, Natriumhydrid. Bevorzugt ist Pyridin oder Triethylamin.

Phosphorodichloridat (VII), Alkohol $R^1$-OH (VIII) und Base verhalten sich wie äquimolar zueinander.

Das in dieser erfindungsgemäßen Weise synthetisierte Monochloridat (IV) wird nach üblichem Verfahren aufgearbeitet, getrocknet, isoliert und am besten ohne weitere Reinigung sofort zur Reaktion gemäß (III) eingesetzt.

Diese Phosphorylierungsreaktion gemäß (III) kann mit ungeschützten oder nur partiell geschützten Zuckern durchgeführt werden, wenn 6-Phosphoryl-Hexose oder 5-Phosphorylpentose hergestellt werden soll, da man Reaktionsbedingungen finden kann, wo selektiv die primäre OH-Funktion des Zuckers mit Phosphorochloridat (IV) in Reaktion tritt. Bei entsprechend eingehaltenen Reaktionsbedingungen kann man auf Blockierung sekundärer Hydroxygruppen des Saccharids verzichten. Der Einsatz des bis auf eine Hydroxygruppe vollständig geschützten Zuckers ist dennoch empfehlenswert. Es ist dann zwingend notwendig, wenn die Phosphatesterfunktion an eine sekundäre Zuckerhydroxygruppe eingeführt werden soll.

Die Reaktion des Formelschemas (III) bedarf Feuchtigkeitsausschlußmaßnahmen.

In einer bevorzugten Ausführung läßt sich diese Phosphorylierung in einem organischen aprotischen Lösungsmittel durchführen. Die Wahl des Lösungsmittels richtet sich unter anderem nach dem einzusetzenden Zucker, der sich darin lösen muß. Bevorzugte Lösungsmittel sind Dichlormethan, Dichlorethan, Tetrahydrofuran, Dioxan und Dimethylformamid.

Die Reaktionstemperatur kann zwischen -40°C und 100°C eingestellt werden. Wenn eine selektive Phosphorylierungsreaktion mit freien oder partiell geschützten Zuckern beabsichtigt ist, wird die niedrige Reaktionstemperatur unterhalb von 0°C erwünscht. Bevorzugte Reaktionstemperatur liegt zwischen -40° und 10°C.

Die Reaktion gemäß (III) erfordert ebenso eine Base als Säurefänger wie die Reaktion gemäß (VI). Für die Wahl der Base gilt das dort erläuterte Kriterium.

Bei der Reaktion (III) wird Phosphorochloridat (IV) mit der Saccharidkomponente (V) im Unterschuß bei Gegenwart einer im Überschuß eingesetzten Base umgesetzt. Der Reaktionsverlauf wird nach dem in der Kohlenhydratchemie üblichen Verfahren dünnschichtchromatographisch verfolgt. Das so erhaltene erfindungsgemäße Phosphotriester (I) wird nach üblichem Verfahren aufgearbeitet und chromatographisch an Kieselgel gereinigt.

Phosphorsäuretriester des Typs Glycosylphosphat

Das erfindungsgemäße Verfahren zur Herstellung läßt sich ebenso gut für die Verbindungsklasse der Glycosylphosphate anwenden, wobei $R^3OH$ geschützte reduzierende Saccharide darstellt.

In einem Alternativverfahren gelangt man zu dieser Substanzklasse, indem Glycosylhalogenide (X), vornehmlich Chloride und Bromide, mit Phosphorsäureester oder Silbersalz der Struktur (XI) umgestzt werden.

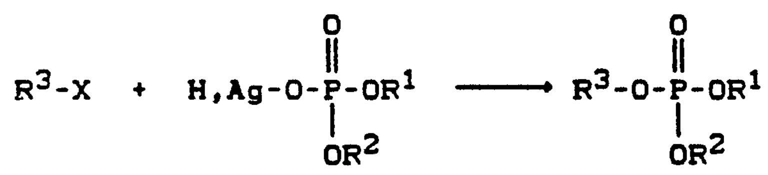 (XII)

(X) (XI) (I)

$R^3$-X is Glycosylhalogenid, X = F,Cl, Br, J,; $R^1$, $R^2$ sind ausgeführt bei der Formel (I).

Diese Reaktion wird unter den für Glycosidsynthesen üblichen Bedingungen durchgeführt. Dort häufig verwendeten Silber-oder Quecksilbersalz-Katalysatoren finden bei dieser Reaktion (XII) ebenso Verwendung. Bevorzugte Katalysatoren sind $Ag_2CO_3$, $AgClO_4$, $AgO_3SCF_3$, $Hg(CN)_2$ und $HgBr_2$. Man kann die Reaktion (XII) in üblichen aprotischen Lösungsmitteln, vorzugsweise Toluol, Dichlormethan, Dichlorethan, oder Acetonitril durchführen. Die Reaktionstemperatur liegt zwischen -50° und 100°C, vorzugsweise zwischen -20° und 50°C. Der Reaktionsverlauf wird nach dem in der Kohlenhydratchemie üblichen Verfahren dünnschichtchromatographisch verfolgt. Das entstandene Glycosylphosphat (I) wird nach üblichen Methoden aufgearbeitet, und an Kieselgel chromatographiert. Das bei der Reaktion (XII) häufig anfallende anomere Gemisch der Saccharidphosphate läßt sich nach üblichen Verfahren in die einzelnen Komponenten zerlegen. Diese Glycosylphosphate sind in anomeren-reiner Form und als Gemisch der beiden Anomeren Gegenstand der Erfindung.

Die für die vorstehend erläuterte Reaktion (XII) einzusetzenden Glycosylhalogenide sind in der Kohlenhydratchemie bekannte Verbindungen. Beispielhaft seien genannt:

Acetobromglucose, -galactose, -mannose, 2,3,4,6-Tetra-O-benzyl-D-gluco-, -galacto-, -manno-pyranosylchlorid, 2-Acetamido-3,4,6-tri-O-acetyl-2-desoxy-α-D-glucopyranosylchlorid, -bromid, 3,4,6-Tri-O-acetyl-2-azido-2-desoxy-D-gluco-, -galacto-oder -manno-pyranosylbromid.

<u>Entblockierung der Phosphatschutzgruppen</u>

Phosphorsäurediester werden aus den oben erläuterten erfindungsgemäßen Verbindungen hergestellt, indem Schutzgruppen $R^2$ durch übliche Deblockierungsreaktionen wie Hydrogenolyse, Dehydrohalogenierung, Reduktion oder alkalische Verseifung abgespalten werden. Auf diese Weise gelangt man zu der erfindungsgemäßen Verbindung des Types (I) mit $R^2$ = H oder Kation.

<u>Entblockierung am Saccharidteil</u>

Saccharidderivate der Verbindungsklassen von Phosphorsäuredi-und -triestern, bei denen ein teilweise geschützter Saccharidteil oder Saccharidbaustein in entblockierter Form vorliegt, werden wie folgt dargestellt.

Übliche Synthesewege gehen von einem völlig geschützten Zucker aus. Man spaltet die Schutzgruppe am Saccharidteil ab und gelangt über einen teilweise entblockierten Saccharidester der Phosphorsäure - schließlich zu Phosphordi-und -triestern der entblockierten Saccharide.

Man kann die Entblockierungsreaktion am Zuckerteil vor, gleichzeitig mit oder nach der Enblockierung an der Phosphatgruppe vornehmen, wenn Phosphodiester der Saccharide angestrebt werden. Bevorzugte Verfahrensweise ist es, daß die endgültige Esterform der Phosphorsäure zuerst vorgefertigt wird und sich dann die Entblockierungsreaktion am Saccharidbaustein anschließt.

Die oben beschriebenen Schutzgruppen werden nach den in der Kohlenhydratchemie üblichen Methoden entfernt.

Liegen z.B. sauer abspaltbare Schutzfunktionen am Zuckerteil vor, wie Acetal-oder Ketalgruppen, werden sie nach üblichen Entblockierungsverfahren der Kohlenhydratchemie (Protective Groups in Organic Chemistry, J.F.W. McOmie, Plenum Press 1973) abgespalten. Isopropyliden, Ethyliden oder Benzyliden geht in einem wäßrigen Medium von Mineralsäure, Essigsäure oder Trifluoressigsäure bei der Temperatur von -40° bis 100°C in einer Reaktionsdauer bis zu 48 Stunden ab. Bevorzugte Reaktionsweise ist eine halbstündige bis fünfstündige Umsetzung in 50 - 90%iger wäßriger Trifluoressigsäure bei -20° bis 25°C.

Acylschutzgruppen wie Acetate oder Benzoate werden unter basischen Bedingungen mit Ammoniak in Alkohol, methanolischer Alkalilauge, Triethylamin in Alkohol oder Natriummethanolat in Methanol bei Temperaturen von -40° bis 50°C innerhalb von bis zu 48 Stunden verseift. Vorzugsweise werden die erfindungsgemäßen Zuckerderivate der Phosphormono-, -di-, oder -triester mit Natriummethanolat in Methanol bei der Reaktionstemperatur von -10° bis 30°C in einer Reaktionszeit bis zu fünf Stunden entblockiert.

Methoxybenzyl, Benzyl, Benzhydryl oder Benzyloxycarbonyl wird nach üblichen Verfahren hydrogenolytisch an einem Pd-C Kontakt abgespalten. Die Hydrogenolytisch wird in einem Lösungsmittel oder -Gemisch von Essigsäure, Alkohol, Tetrahydrofuran, Dioxan, Wasser, oder Mineralsäure durchgeführt und dauert bis zu 7 Tagen.

Die auf die oben ausgeführten Weisen entblockierten Saccharid-phosphorsäureester werden nach dem jeweiligen üblichen Verfahren aufgearbeitet und im Fall von Phosphorsäureestern mit einer oder zwei sauren OH-Gruppen in die saure Form oder Salzform überführt. Im letzteren Fall wird die Neutralisation der sauren Phosphorsäurediester mit der entsprechenden Base durchgeführt und das Salz gefriergetrocknet, kristallisiert oder durch Gelfiltration gereinigt.

Saccharidphosphate und ihre Ester sind in der tierischen und pflanzlichen Welt weit verbreitet. Von großer biologischen Bedeutung sind die im Zuckerstoffwechsel auftretenden Phosphate einzelner Monosaccharide. In der Biosynthese der Glycoproteine und Proteoglycane beispielsweise fungieren außerdem Saccharidphosphat-oder -diphosphatester der langkettigen und lipophilen Polyisoprenoidalkohole als lipidgebundene Oligosaccharidvorstufen. Da Glycoproteine und Proteoglycane integrale Bestandteile der Zellmembranen von Viren, Pilzen und

Mikroorganismen sind, ist die Hemmung der Glycoprotein-Biosynthese ein aussichtsreiches Bekämpfungsprinzip solcher pathogenen Lebewesen.

Die erfindungsgemäßen Verbindungen sind Analoga der natürlich vorkommenden Zuckerphosphate und ihrer Ester und inhibieren in vitro die Glycoproteinbiosynthese. Sie stellen für die Bekämpfung der Viren, Mikroorganismen und Pilze interessante Verbindungen dar.

Als Analoga der Polyisoprenylphosphate greifen die erfindungsgemäßen Verbindungen außerdem in den Lipidstoffwechsel ein und senken in vivo den Serumcholesterin-Spiegel. Die Verbindung des Beispiels 21 Beispielsweise, appliziert im Futter in einer Konzentration von 600 mg/kg Futter, reduziert den Cholesterinwert im Serum von ca. 350 g schweren, genetisch hypercholesterinämischen Ratten signifikant um mehr als 10 % nach 7-tägiger Applikation.

Auf der anderen Seite sind die erfindungsgemäßen Verbindungen für Tiere verträglich. So ist die $LD_{50}$ von der erfindungsgemäßen Verbindung des Beispiels 21 bzw. 25 größer als 2000 mg/kg per os. Als Versuchstiere dienten hierbei ungefähr 100 g schwere männliche Ratten.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll-und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium-und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxide oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Gylcerin, Glycerinformal, Tetrahydrofurfurylalkohol, Poly ethylenglykole und Fettsäureester des Sorbitans oder Gemische dieser Stoffe enthalten.

Zur parenteralen Applickation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit-und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs-und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Me thoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human-als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können Tieren in den üblichen Konzentrationen und Zubereitungen zusammen mit dem Futter bzw. mit Futterzubereitungen oder mit dem Trinkwasser gegeben werden.

Beispiel 1

(1,2:3,4-Di-O-isopropyliden-α-D-galactopyranose-6-yl)methyl-phenylphosphorsäureester

Phenylphosphorodichloridat (10 ml, 67 mmol), gelöst in trockenem n-Hexan (150 ml), wird bei 0°C tropfenweise mit einer Lösung von Methanol (2,7 ml, 67 mmol) und abs. Pyridin (5,4 ml, 67 mmol) in trockenem n-Hexan (150 ml) versetzt. Nach der 40 minütigen Tropfzeit wird der Ansatz noch eine Stunde bei 0°C gerührt, dann filtriert und das Filtrat eingeengt. Der zurückbleibende Sirup wird anschließend bei RT in abs. Dichlormethan (100 ml) gelöst und mit 1,2:3,4-Di-O-isopropyliden-α-D-galactopyranose (12,0 g, 46 mmol) und abs. Pyridin (10 ml) versetzt. Nach der vollständigen Umsetzung wird der Ansatz filtriert, eingeengt und säulenchromatographisch (Toluol : Aceton 5:1) gereinigt, Ausbeute 13,9 g (70 %).
Rf (Toluol : Aceton 3:1) 0,54
$^1$H-NMR (360 MHZ, $CDCl_3$): δ = 1.33, 1.43, 1.44, 1.49,

1.54
(s, 12 H, 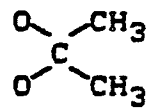 )

3.89 (zwei d, $J_{P,CH_3}$ = 11.3 Hz, 3H, $OCH_3$), 4.11 (m, 1H, 5-H), 4.63 (zwei dd, $J_{2,3}$ = 2.8 Hz, $J_{3,4}$ = 7.8 Hz, 1H, 3-H), 5.57 (zwei d, $J_{1,2}$ = 5.0 Hz, 1H, 1-H), 7.15-7.40 (m, 5H, Ph).
$C_{19}H_{27}O_9P$ (430.4) Ber. C 53.0, H 6.3
Gef. C 52.9, H 6.1

Beispiel 2

(1,2:3,4-Di-O-isopropyliden-α-D-galactopyranose-6-yl)ethylphenylphosphorsäureester

Diese Verbindung wird nach dem Verfahren des Beispiels 1 mit Ethanol (3.9 ml, 67 mmol) statt Methanol hergestellt, Ausbeute 11.2 g (55 %).
Rf (Toloul : Aceton 2:1) 0.52
$^1$H-NMR (360 MHz, $CDCl_3$): δ = 1.37 (t, $J_{CH_2}$, $CH_3$ = 7,0 Hz, 3H, $CH_3$), 1.34, 1.45, 1.50, 1.55 (s, 12 H,

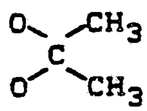 )

4.12 (m. 1H, 5-H), 4.63 (zwei dd, $J_{2,3}$ = 2.6 Hz, $J_{3,4}$= 7.7 Hz, 1H, 3-H), 5.56 (d, $J_{1,2}$ = 4.9 Hz, 1H, 1-H), 7.13-7.38 (m, 5H, Ph).
$C_{20}H_{29}O_9P$ (444.4) Ber. C 54.1, H 6.6
Gef. C 54.0, H 6.6

Beispiel 3

(1,2:3,4-Di-O-isopropyliden-α-D-galactopyranose-6-yl)isopropylphenylphosphorsäureester

Diese Verbindung wird nach dem Verfahren des Beispiels 1 mit Isopropanol (5,0 ml, 67 mmol) statt Methanol hergestellt, Ausbeute 6,5 g (31 %).
Rf (Toluol : Aceton 3:1) 0.50
$^1$H-NMR (360 MHz, $CDCl_3$): δ = 1.29-1.39 (m, 6H,

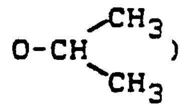

1.32, 1.43, 1.48, 1.51 (s, 12H,

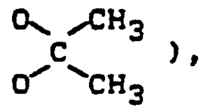

4.09 (m, 1H, 5-H), 4.61 (m, 1H, 3-H), 4.73-4.88 (m, 1H, 0-CH), 5.54 (d, $H_{1,2}$ = 5,0 Hz, 1H, 1-H), 7.13-7.38 (m, 5H, Ph).
$C_{21}H_{31}O_9P$ (458.4) Ber. C 55.0, H 6.8
Gef. C 54.9, H 6.8

Beispiel 4

n-Butyl(1,2:3,4-Di-O-isopropyliden-α-D-galactopyranose-6-yl)phenylphosphorsäureester

Diese Verbindung wird nach dem Varfahren des Beispiels 1 mit n-Butanol (4.9 ml, 67 mmol) statt Methanol hergestellt, Ausbeute 14.8 g (68 %).
Rf (Toluol : Aceton 3:1) 0.61
$^1$H-NMR (360 MHz, $CDCl_3$): δ = 0.91 (t, $J_{CH_2, CH_3}$ = 7.0 Hz, 3H, $-CH_2-C\underline{H}_3$), 1.30-1.45 (m, 2H, $-C\underline{H}_2-CH_3$), 1.32, 1.44, 1.49, 1.53 (s, 12H,

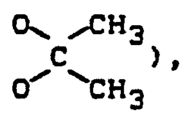

1.67 (quintett, 2H, $-OCH_2C\underline{H}_2$), 4.10 (m, 1H, 5-H), 4.63 (zwei dd, $J_{2,3}$ = 2.6 Hz, $J_{3,4}$ = 7.8 Hz, 1H, 3-H), 5.54 (d, $J_{1,2}$ = 5.0 Hz, 1H, 1-H), 7.15-7.38 (m, 5H, Ph).
$C_{22}H_{33}O_9P$ (472.5) Ber. C 55.9, H 7.0
Gef. C 55.9, H 6.9

Beispiel 5

(1,2:3,4-Di-O-isopropyliden-α-D-galactopyranose-6-yl)isobutylphenylphosphorsäureester

Diese Verbindung wird nach dem Verfahren des Beispiels 1 mit Isobutanol (4,9 ml, 67 mmol) statt Methanol hergestellt, Ausbeute 7,8 g (36 %).
Rf (Toluol : Aceton 3:1) 0,61
$C_{22}H_{33}O_9P$ (472.5) Ber. C 55.9, H 7.0
Gef. C 55.9, H 6.9

Beispiel 6

(1,2:3,4-Di-O-isopropyliden-α-D-galactopyranose-6-yl)phenyltetradecylphosphorsäureester

Diese Verbindung wird nach dem Verfahren des Beispiels 1 mit Tetradecanol (14 g, 67 mmol) statt Methanol hergestellt. Die säulenchromatographische Reinigung wird mit dem Laufmittel Toluol : Aceton 15:1 durchgeführt, Ausbeute 18,3 g (65 %)
Rf (Toluol : Aceton 3:1) 0,63, $[\alpha]_D^{20}$ = -27,8° (C = 1,47, Aceton).
$^1$H-NMR (360 MHz, $CDCl_3$): δ = 0.89 (t, $J_{CH_2}$, $CH_3$ = 7.0 Hz, 3H, -$CH_2C\underline{H}_3$), 1.27 (S, 22H, -$(CH_2)_{11}$-Me), 1.30, 1.33, 1.34, 1.44, 1.45, 1.49, 1.54 (s, 12H,

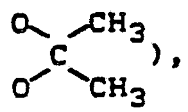

1.70 (m, 2H, $OCH_2C\underline{H}_2$), 4.34 (dd, $J_{1,2}$ = 4.9 Hz, $J_{2,3}$ = 2.5 Hz, 1H, 2-H), 4.63 (zwei dd, $J_{3,4}$ = 7.9 Hz, 1H, 3-H), 5.55 (d, 1H, 1-H), 7.16-7.38 (m, 5H, Ph).
$C_{32}H_{52}O_9P$ (612.0) Ber. C 62.7, H 8.7, P 5.1
Gef. C 62.7, H 8.5, P 4.9

Beispiel 7

(1,2:3,4-Di-O-isopropyliden-α-D-galactopyranose-6-yl)octadecylphenylphosphorsäureester

Diese Verbindung wird nach dem Verfahren des Beispiels 1 mit Octadecanol (18,0 g, 67 mmol) statt Methanol her gestellt. Die säulenchromatographische Reinigung wird mit dem Laufmittel Toluol : Aceton 9:1 durchgeführt, Ausbeute 26,1 g (61 %).
Rf (Toluol : Aceton 3:1) 0,65
$^1$H-NMR (360 MHz, $CDCl_3$): δ = 0.89 (t, $J_{CH_2}$, $CH_3$ = 7.0 Hz, 3H, -$CH_2C\underline{H}_3$), 1.27 (S, 30H, -$(CH_2)_{15}$-Me), 1.30, 1.33, 1.34, 1.44, 1.45, 1.49, 1.54 (s, 12H,

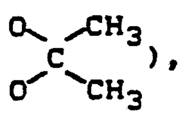

1.70 (m, 2H, $OC\underline{H}_2CH_2$), 4.34 (dd, $J_{1,2}$ = 4.9 Hz, $J_{2,3}$ = 2.5 Hz, 1H, 2-H), 4.63 (zwei dd, $J_{3,4}$ = 7.9 Hz, 1H, 3-H), 5.55 (d, 1H, 1-H), 7.16-7.38 (m, 5H, Ph).
$C_{35}H_{61}O_9P$ (668.8) Ber. C 64.6, H 9.2
Gef. C 64.6, H 9.1

Beispiel 8

Cyclopentyl (1,2:3,4-di-O-isopropyliden-α-D-galactopyranose-6-yl)phenylphosphorsäureester

Diese Verbindung wird nach dem Verfahren des Beispiels 1 mit Cyclopentanol (6,0 ml, 67 mmol) statt Methanol hergestellt, Ausbeute 6,7 g (30 %).
Rf (Toluol : Aceton 5:1) 0,42.
$^1$H-NMR (360 MHz, $CDCl_3$): δ = 1.25-1.97 (m, 8H,

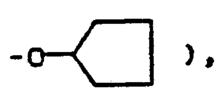

1.32, 1.42, 1.48, 1.52 (s, 12H,

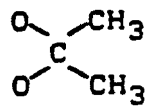 ), 4.09

(m, 1H, 5-H), 4.33 (dd, $J_{1,2}$ = 5.0 Hz, $J_{2,3}$ = 2.4 Hz, 1H, 2-H), 4.61 (m, 1H, 3-H), 4.96-5.08 (m, 1H,

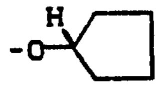 ),

5.54 (d, 1H, 1-H), 7.15-7.36 (m, 5H, Ph).
$C_{23}H_{33}O_9P$ (484.5) Ber. C 57.0, H 6.9
Gef. C 56.9, H 6.9

Beispiel 9

Cyclohexyl(1,2:3,4-di-O-isopropyliden-α-D-galactopyranose-6-yl)phenylphosphorsäureester

Die Verbindung wird nach dem Verfahren des Beispiels 1 mit Cyclohexanol (7,1 ml, 67 mmol) statt Methanol hergestellt, Ausbeute 9,2 g (40 %).
Rf (Toluol : Aceton 3:1) 0,60
$^1$H-NMR (360 MHz, CDC1$_3$): δ = 1.17-2.04 (m, 10H,

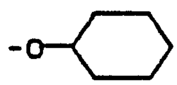 ),

1.32, 1.44, 1.48, 1.53 (s, 12H

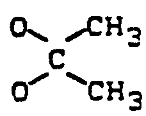 ),

4.11 (m, 1H, 5-H), 4.33 (dd, $J_{1,2}$ = 5.0 Hz, $J_{2,3}$ = 1H, 2-H), 4.43-4.59 (m, 1H,

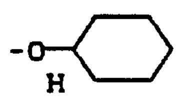

), 4.62 (zwei dd, $J_{3,4}$ = 7.9 Hz, 1H, 3-H), 5.54 (d, 1H, 1-H), 7.13-7.38 (m, 5H, Ph).
$C_{24}H_{35}O_9P$ ( 498.7) Ber. C 57.8, H 7.1
Gef. C 57.8, H 7.1

Beispiel 10

2-Bromethyl(1,2:3,4-di-O-isoproypliden-α-D-galactopyranose-6-yl)phenylphosphorsäureester

Diese Verbindung wird nach dem Verfahren des Beispiels 1 mit 2-Bromethanol (5,6 ml, 67 mmol) statt Methanol hergestellt, Ausbeute 21,0 g (72 %).
Rf (Toluol : Ethanol 10:1) 0.38
$^1$H-NMR (360 MHz, $CDCl_3$): δ = 1.32, 1.43, 1.44, 1.49, 1.54 (s, 12H,

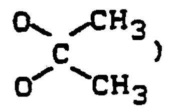

, 3.56 (t, 1H, -O-$CH_2$-$CH_2$-Br), 3.72 (t, 1H, -O-$CH_2CH_2$Br), 4,09 (m, 1H, 5-H), 4.63 (zwei dd, J $_{2,3}$ = 2.4 Hz, $J_{3,4}$ = 7.8 Hz, 1H, 3-H), 5.55 (zwei d, 1H, 1-H), 7.15-7.41 (m, 5H, Ph).

$C_{20}H_{28}BrO_9P$ (523.3) Ber. C 45.9, H 5.4, Br. 15.3, P 5.9

Gef. C 45.7, H 5.4, Br. 15.0, P 6.0

Beispiel 11

Allyl(1,2:3,4-di-O-isopropyliden-α-D-galactopyranose-6-yl)phenylphosphorsäureester

Diese Verbindung wird nach dem Verfahren des Beispiels 1 mit Allylalkohol (4,4 ml, 67 mmol) statt Methanol hergestellt, Ausbeute 14,1 g (68 %).

Rf (Toluol : Aceton 3:1) 0.59.

$^1$H-NMR (360 MHz, $CDCl_3$): $\delta$ = 1.34, 1.44, 1.49, 1.54 (s, 12H,

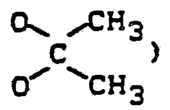

, 4.12 (m, 1H, 5-H), 4.34 (dd, $J_{1,2}$ = 4.8 Hz, $J_{2,3}$ = 2.6 Hz, 1H, 2-H), 4.59-4.73 (m, 3H, 3-H, A11), 5.28 (m, 1H, A11), 5.39 (m, 1H, A11), 5.56 (d, 1H, 1-H), 5.97 (m, 1H, A11), 7.15-7.41 (m, 5H, Ph).

$C_{21}H_{29}O_9P$ (456.4) Ber. C 55.3, H 6.4

Gef. C 55.1, H 6.3

Beispiel 12

n-Butyl-o-chlorphenyl(1,2:3,4-di-O-isopropyliden-α-D-galactopyranose-6-yl)phosphorsäureester

O-Chlorphenylphosphorodichloridat (1,1 ml, 6,7 mmol), gelöst in trockenem n-Hexan (5 ml), wird bei 0°C tropfenweise mit einer Lösung von n-Butanol (0,61 ml, 6.7 mmol) und abs. Pyridin (0,54 ml, 6.7 mmol) versetzt. Nach der 40 minütigen Tropfzeit wird der Ansatz noch eine weitere Stunde bei 0°C gerührt, dann filtriert und das Filtrat eingeengt. Der zurückbleibende Sirup wird anschließend bei RT in abs. Dichlormethan (10 ml) gelöst und mit 1,2:3,4-Di-O-isopropyliden-α-D-galactopyranose (1,16 g, 4,47 mmol) und abs. Pyridin (1 ml) versetzt. Nach der vollständigen Umsetzung wird der Ansatz filtriert, eingeengt und säulenchromatographisch (Toluol : Aceton 5:1) gereingt, Ausbeute 1,47 g (65 %).

Rf (Toluol : Aceton 5:1) 0,58

$^1$H-NMR (360 MHz, $CDCl_3$): $\delta$ = 0,92 (t, $J_{CH_2CH_3}$ = 7.0 Hz, 3H, $CH_2C\underline{H}_3$), 1.30-1.47 (m, 2H, -$C\underline{H}_2CH_3$), 1.32, 1.44, 1.49, 1.52 (s, 12H,

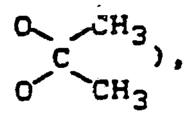

1.70 (m, 2H, -O-$CH_2C\underline{H}_2$), 4.12 (m, 1H, 5-H), 4.34 (dd, $J_{1,2}$ = 5.0 Hz, $J_{2,3}$ = 2.6 Hz, 1H, 2-H), 4.63 (dd, $J_{3,4}$= 8.0 Hz, 1H, 3-H), 5.55 (d, 1H, 1-H), 7.08-7.57 (m, 4H, Ph).

$C_{22}H_{32}ClO_9P$ (506.9) Ber. C 52.1, H 6.4, C1 7.0

Gef. C 52.0, H 6.3, Cl 6.8

Beispiel 13

n-Butyl-p-chlorphenyl(1,2:3,4-di-O-isopropyliden-α-D-galactopyranose-6-yl)phosphorsäureester

Diese Verbindung wird nach dem Verfahren des Beispiels 12 mit p-Chlorphenylphosphorodichloridat (1,09 ml, 6,7 mmol) statt o-Chlorphenylphosphorodichloridat hergestellt, Ausbeute 1.40 g (62 %).
Rf (Toluol : Aceton 5:1) 0.52
$^1H$-NMR (360 MHZ, $CDCl_3$): $\delta$ = 0.91 (t, 3H, $CH_2CH_3$), 1.37 (m, 2H, $-CH_2CH_3$), 1.32, 1.42, 1.47, 1.51 (s, 12H,

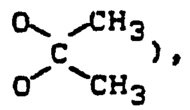

1.67 (quintett, 2H, $-OCH_2CH_2$), 4.07 (m, 1H, 5-H), 4.33 (dd, $J_{1,2}$ = 5.0 Hz, $J_{2,3}$ = 2.4 Hz, 1H, 2-H), 4.63 (dd, $J_{3,4}$ = 7.9 Hz, 1H, 3-H), 5.54 (d, 1H, 1-H), 7.18-7.35 (m, 4H, Ph).
$C_{22}H_{32}ClO_9P$ (506.9) Ber. C 52.1, H 6.4, Cl 7.0
Gef. C 51.9, H 6.3, Cl 6.8

Beispiel 14

n-Butyl(1,2:3,4-di-O-isopropyliden-α-D-galactopyranose-6-yl)p-nitrophenylphosphorsäureester

Diese Verbindung wird nach dem Verfahren des Beispiels 12 mit p-Nitrophenylphosphorodichloridat (1.72 g, 6.7 mmol) statt o-Chlorphenylphosphorodichloridat hergestellt, Ausbeut 0,54 g (23 %).
Rf (Toluol : Aceton 3:1) 0,57
$^1H$-NMR (360 MHz, $CDCl_3$): $\delta$ = 0.94 (t, 3H, $-CH_2CH_3$), 1.25-1.55 (m, 2H, $CH_2Me$), 1.34, 1.44, 1.49, 1.52 (s, 12H

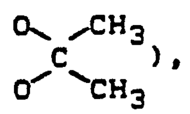

1.72 (quintett, 2H, $OCH_2CH_2$), 4.11 (m, 1H, 5-H), 4.65 (m, 1H, 3-H), 5.55 (d, $J_{1,2}$ = 5.0 Hz, 1H, 1-H), 6.90-8.28 (m, 4H, Ph).
$C_{22}H_{32}NO_{11}P$ (517.5) Ber. C 51.1, H 6.2, N 2.7 Gef. C 50.9, H 6.1, N 2.6

Beispiel 15

(1,2:5,6-Di-O-isopropyliden-α-D-glucofuranose-3-yl)phenyltetradecylphosphorsäureester

Diese Verbindung wird nach dem Verfahren des Beispiels 6 mit 1,2:5,6-Di-O-isopyropyliden-α-D-gluocfuranose (12,0 g, 46 mmol) statt 1,2:3,4-Di-O-isopropyliden-α-D-galactopyranose hergestellt und säulenchromatographisch (Toluol : Essigester 10:1) gereinigt, Ausbeute 6,9 g (24 %).
Rf (Toluol : Essigester 10:1) 0,46
$^1H$-NMR (360 MHz, $CDCl_3$): $\delta$ = 0,87 (t, 3H, $CH_2CH_3$), 1.20-1.39 (s, 22H, $-(CH_2)_{11}-CH_3$), 1.27, 1.29, 1.30, 1.40, 1.49, 1.50 (s, 12H,

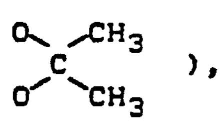

1.69 (m, 2H, $OCH_2$-$CH_2$), 4.61, 4.83 (d, 1H, 3-H), 4.93 (m, 1H, 2-H), 5.79, 5.94 (d, $J_{1,2}$ = 3.7 Hz, 1H, 1-H), 7.16-7.41 (m, 5H, Ph).

$C_{33}H_{53}O_9P$ (612.7) Ber. C 62.7, H 8.7
Gef. C 62.7, H 8.7

## Beispiel 16

Phenyl(2,3, 4,6-tetra-O-acetyl-β-D-glucopyranosyl)tetradecylphosphorsäureester

Phenyltetradecylphosphorsäureester (11,3 g, 30,6 mmol) und Silbercarbonat (10,0 g, 36,3 mmol) werden unter Stickstoffatmosphäre und Lichtschutz in abs. Acetonitril (100 ml) vorgelegt. Dazu tropft man bei Raumtemperatur eine Lösung von Acetobromglucose (10,4 g, 25,3 mmol) in abs. Acetonitril (100 ml). Nach Beendigung der Reaktion wird der Ansatz filtriert, eingeengt und säulenchromatographisch (Toluol:Essigester 2:1) gereinigt, Ausbeute: 9,4 g (54 %).
$^1$H-NMR (360 MHz, $CDCl_3$): δ = 0.88 (t, 3H, $CH_2CH_3$), 1.25 (s, 22H, $(CH_2)_{11}$-$CH_3$), 1.67 (m, 2H, $OCH_2CH_2$), 2.03, 2.05, 2.09, 2.10, 2.11, 2.20, (s, 12H, CO-$CH_3$), 4.94 (dd, $J_{1,2}$ = 3.8 Hz, $J_{2,3}$ = 10.1 Hz, 1H, 2-H), 5.11 (dd, $J_{4,5}$ = 10.0 Hz, $J_{3,4}$ = 9.4 Hz, 1H, 4-H), 5.14 (dd, $J_{4,5}$ = 10.0 Hz, $J_{3,4}$ = 9.6 Hz, 1H, 4-H), 5.30 (dd, 1H, 3-H), 5.57 (dd, 1H, 3-H), 5.49 (d, 1H, 1-H), 6.27 (d, 1H, 1-H), 7.16-7.36 (m, 5H, Ph).

$C_{34}H_{53}O_{13}P$ (700.7) Ber. C 58.3, H 7.6
Gef. C 58.2, H 7.6

## Beispiel 17

(1,2:3,4-Di-O-isopropyliden-α-D-galactopyranose-6-yl)methylphosphorsäureester

Platinoxid (10 g) wird in Eisessig (150 ml) unter Wasserstoffnormaldruck hydriert. Zu dieser Platinsuspension wird dann eine Lösung von Produkt des Beispiels 1 (10 g) in abs. Dichlormethan (200 ml) gegeben und weiter hydriert. Nach Beendigung der Hydrierung wird der Katalysator abfiltriert und das Filtrat eingeengt, Ausbeute 7,8 g (95 %), $[\alpha]_D^{20}$ = 56,1° (C = 0,26, Aceton).
Rf (n-Butanol : Essigsäure : Wasser 10:1:1) 0,29
$C_{13}H_{23}O_9P$(354,3) Ber. C 44.1 H 6.5
Gef. C 44.1 H 6.5

## Beispiel 18

Ethyl(1,2:3,4-di-O-isopropyliden-α-D-galactopyranose-6-yl)-phosphorsäureester

Das im Beispiel 2 beschriebene Produkt wird nach dem Verfahren des Beispiels 17 hydriert, Ausbeute 7,79 g (94 %), $[\alpha]_D^{20}$ = 42,7° (C = 0,78, $CHCl_3$)
$^1$H-NMR (360 MHz, $CDCl_3$): δ = 1.34 (t, 3H, -$CH_2CH_3$), 1.34, 1.44, 1.45, 1.55 (s, 12H

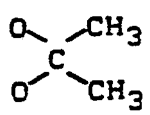
),

4.04-4.24 (m, 5H, 5-H, 6-H, 6'-H, $OCH_2$), 4.31 (zwei dd, $J_{3,4}$ = 7.9 Hz, $J_{4,5}$ = 1.8 Hz, 1H, 4-H), 4.32 (zwei dd, $J_{2,3}$ = 2.4 Hz, $J_{1,2}$ = 5.0 Hz, 1H, 2-H), 4.64 (zwei dd, 1H, 3-H), 5.54 (zwei d, 1H, 1-H), 6.75 (s breit, 1H, OH).
$C_{14}H_{25}O_9P$ (368.3) Ber. C 45.7, H 6.8
Gef. C 45.7, H 6.8

## Beispiel 19

n-Butyl(1,2:3,4-di-O-isopropyliden-α-D-galactopyranose-6-yl)phosphorsäureester

Das in Beispiel 4 beschriebene Produkt wird nach dem Verfahren des Beispiels 17 hydriert, Ausb. 7.72 g (92%).
$[\alpha]_D^{20}$ = -22.2° (C = 0.40, $CHCl_3$)
Rf (n-Butanol: Essigsäure: Wasser 10:1:1) 0.34
$C_{16}H_{29}O_9P$(396.4) Ber. C 48.5 H 7.4 Gef C 48.5 H 7.3

Beispiel 20

2-Bromethyl (1,2:3,4-di-O-isopropyliden-α-D-galactopyranose-6-yl)phosphorsäureester

Das im Beispiel 10 beschriebene Produkt wird nach dem Verfahren des Beispiels 17 hydriert, Ausb. 7.95 g (93%).
$[\alpha]_D^{20}$ = 56.4° (C = 0.72, $CHCl_3$)
Rf (Essigester: Essigsäure:Pyridin:Wasser 10:2:2:1) 0.50
$^1$H-NMR(360MHz, $CDCl_3$):$\delta$ = 1.34, 1.45, 1.55 (s,12H,

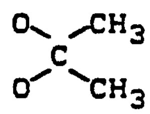
).

3.57(t,1H, $OCH_2CH_2Br$), 3.74(t,1H, $OCH_2CH_2Br$), 4.07 (ddd, $J_{5,6}$ = 6.2Hz, $J_{5,6}$ = 6.2Hz, 1H,5-H), 4.11-4,23 (m, 2H, 6-H, 6'-H),
4.23-4.38 (m, 2H, $OCH_2CH_2Br$), 4.30 (dd, $J_{3,4}$ = 7.9Hz, $J_{4,5}$ = 1.7Hz, 1H, 4-H), 4.34 (dd, $J_{1,2}$ = 4.9Hz, $J_{2,3}$ = 2.4Hz, 1H, 2-H), 4.64(dd, 1H,3-H), 5.55 (d, 1H, 1-H), 6.55 (sbreit, 1H, OH).
$C_{14}H_{24}BrO_9P$ (447.2) Ber. C 37.6 H 5.4 Br 17.9
Gef. C 37.5 H 5.3 Br 17.3

Beispiel 21

(1,2:3,4-Di-O-isopropyliden-α-D-galactopyranose-6-yl)tetradecylphosphorsäureester

Das im Beispiel 6 beschriebene Produkt wird nach dem Verfahren des Beispiels 17 hydriert, Ausb. 8.32 g (95%).
Rf (n-Butanol:Essigsäure:Wasser 10:1:1) 0.44
$[\alpha]_D^{20}$ = 33.5° (C = 1.03, $CHCl_3$)
$^1$H-NMR (360 MHz, $CDCl_3$): $\delta$ = 0.88 (t, 3H, $CH_2C\underline{H}_3$), 1.22-1.39 (s breit, 22H, $-(C\underline{H}_2)_{11}CH_3$), 1.32, 1,33, 1.44, 1.54 (s, 12H,

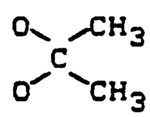
)

1.65 (m, 2H, $OCH_2C\underline{H}_2$), 4.00 (td, 2H, O-$CH_2$), 4.04-4.21 (m, 3H, 5-H, 6-H, 6'-H), 4.29 (dd, $J_{3,4}$ = 7.8Hz, $J_{4,5}$-1.6Hz, 1H, 4-H), 4.33 (dd, $J_{2,3}$ = 2.4Hz, 1H, 2-H), 4.63(dd, 1H, 3-H), 5.53 (d, 1H, 1-H), 7.43 (s breit, 1H, OH)
$C_{26}H_{49}O_9P$ (536.6) Ber. C 58.2 H 9.2
Gef. C 58.2 H 9.1.

Beispiel 22

(1,2:3,4-Di-O-isopropyliden-α-D-galactopyranose-6-yl)octadecylphosphorsäureester

Das im Beispiel 7 beschriebene Produkt wird nach dem Verfahren des Beispiels 17 hydriert, Ausb. 7.94 g (90%).
Rf (n-Butanol:Essigsäure:Wasser 10:1:1) 0.46
$[\alpha]_D^{20}$ = 37.4° (C=0.23, $CHCl_3$)
$^1$H-NMR (360 MHz, $CDCl_3$): δ=0.88 (t,3H, $CH_2C\underline{H}_3$), 1.22-1.39 (s breit, 30H,-$(C\underline{H}_2)_{15}CH_3$), 1.32, 1.33, 1.44, 1.54 (s, 12H,

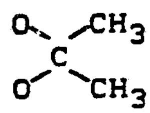 )

1.65 (m, 2H, $OCH_2C\underline{H}_2$), 4.00 (td, 2H, O-$CH_2$), 4.04-4.21 (m, 3H, 5-H, 6-H, 6'-H), 4.29(dd, $J_{3,4}$=7.8Hz, $J_{4,5}$=1.6Hz, 1H, 4-H), 4.33 (dd, $J_{2,3}$=2.4Hz, 1H,2-H), 4.63(dd, 1H, 3-H), 5.53(d,1H,1-H), 7.43 (s breit, 1H, OH)
$C_{30}H_{57}O_9P$(592.7) Ber. C 60.8 H 9.7
Gef. C 60.8 H 9.6.

Beispiel 23

(1,2:5,6-Di-O-isopropyliden-α-D-glucofuranose-3-yl)tetradecylphosphorsäureester

Das im Beispiel 18 beschriebene Produkt wird nach dem Verfahren des Beispiels 17 hydriert, Ausb. 8.21 g (91%).
Rf (n-Butanol:Essigsäure:Wasser 10:1:1) 0.42
$[\alpha]_D^{20}$ = 5.9° (C=1.035, $CHCl_3$)
$C_{26}H_{49}O_9P$ (536.6) Ber. C 58.2 H 9.2
Gef. C 58.2 H 9.1.

Beispiel 24

(2,3,4,6-Tetra-O-acetyl-β-D-gluocopyranosyl)tetradecylphosphorsäureester

Das im Beispiel 16 beschriebene Produkt wird nach dem Verfahren des Beispiels 17 hydriert, Ausb. 6.8 g (75%). $^1$H-NMR (360 MHz, $CDCl_3$): δ=0.85 (t, 3H, $CH_2C\underline{H}_3$), 1.34-1.37(s breit, 22H, $(C\underline{H}_2)_{11}$-$CH_3$), 1.64 (G, 2H, $OCH_2C\underline{H}_2$)m 2.00, 2.02, 2.05, 2.07 (s, 12H, CO-$CH_3$), 3.84 (ddd, $J_{5,6}$ =4.2Hz, $J_{5,6'}$=2.2Hz, $J_{4,5}$=9.9Hz, 1H, 5-H), 4.19 (dd, $H_{6,6'}$=12.5 Hz, 1H, 6'-H), 4.28 (dd, 1H, 6-H), 5.08 (dd, $J_{1,2}$=7.8 Hz, $J_{2,3}$=9.4 Hz, 1H, 2-H), 5.14 (dd, $J_{3,4}$=9.4Hz, 1H, 4-H), 5.23 (dd, $J_{p,1}$ = 7.8 Hz, 1H, 1-H), 5.26 (dd, 1H, 3-H), 7.78 (s breit, 1H, OH).
$C_{28}H_{49}O_{13}P$ (642.6) Ber. C 52.3 H 7.7
Gef. C 52.2 H 7.6.

Beispiel 25

D-Galactopyranose-6-yl-tetradecyl-phosphorsäureester

Das im Beispiel 21 beschriebene Produkt (4.3 g, 9.4 mmol) wird bei 0°C in 90 %iger wäßriger Trifluoressigsäure (40 ml) gelöst und nach einer Stunde an HV eingeengt. Der Rückstand wird dreimal mit Toluol versetzt und das Toloul wieder abdestilliert.
Ausb. 3.86 g (90 %), Schmp. 119-120°C, $[\alpha]_D^{20}$ = +11.3° (C = 1.30, DMF).
$C_{20}H_{41}O_9P$(456.5) Ber. C 52.6 H 9.l
Gef. C 52.5 H 9.0

Beispiel 26

2-Bromethyl-D-galactopyranose-6-yl-phosphorsäureester

Das im Beispiel 20 beschriebene Produkt (1.0 g, 2.24 mmol) wird nach dem Verfahren des Beispiels 25 hydrolysiert. Ausb. 750 mg (91 %),
Rf (Dichlormethan:Methanol:Wasser 10:10:3) 0.38
$^1$H-NMR(360 MHz, $D_2O$): $\delta = 4.49$ (d,$J_{1,2} = 7.9$ Hz, 1-H$\beta$), 5.15(d,$J_{1,2} = 3.8$ Hz, 1-H$\alpha$)
$C_8H_{16}BrO_9P$(367.1) Ber. C 26.2 H 4.4 Br 21.8
Gef. C 26.1 H 4.4 Br 21.6

Beispiel 27

($\beta$-D-Glucopyranosyl)-tetradecylphosphorsäureester

Das im Beispiel 24 beschriebene Product (72 mg, 0.11 mmol) wird in abs. Methanol (2 ml) gelöst und mit einer 0.2N-Lösung von Natriummethanolat in Methanol (2 ml) versetzt. Nach einer Stunde wird die Reaktionslösung mit Ionenaustauscher Dowex 50Wx4($H^+$) neutralisiert, filtriert und gefriergetrocknet, Aus. 44 mg (82 %).
Rf (Essigester:Essigsäure:Pyridin:Wasser 10:2:2:1) 0.25
$C_{20}H_{40}NaO_9P$(478.5) Ber. C 50.2 H 8.4
Gef. C 50.1 H 8.4

**Ansprüche**

1. Verbindungen der allgemeinen Formel (I)

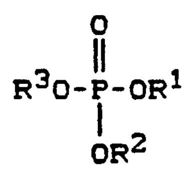

(I)

in welcher
$R^1$ einen gegebenenfalls substituierten geradkettigen oder verzweigten gesättigten oder einfach oder mehrfach ungesättigten Alkylrest oder einen gegebenenfalls substituierten, gesättigten oder einen ein-oder mehrfach ungesättigten Cycloalkylrest,
$R^2$ Wasserstoff, Phosphatschutzgruppen oder ein anorganisches oder organisches Kation bedeuten und
$R^3$ einen freien oder geschützten Saccharidrest in der Pyranose-oder Furanoseform aus der Reihe der Hexosen oder Pentosen bedeutet.

2. Verbindungen nach Anspruch 1, dadurch gekennzeichnet, daß
$R^1$ für Alkyl oder Alkenyl oder Alkinyl mit bis zu 40 C-Atomen, gegebenenfalls ein-oder mehrfach ungesättigtes Cycloalkyl mit 3 bis 7 C-Atomen, $C_6$-$C_{10}$-Ar-$C_1$-$C_{40}$-alkyl, $C_6$-, $C_{10}$-oder $C_{12}$-Aryl und
$R^2$ für $C_6$-, $C_{10}$-oder $C_{12}$-Aryl, Phosphatschutzgruppen, Wasserstoff oder ein anorganisches oder organisches Kation stehen.

3. Verbindungen nach den Asnprüchen 1 und 2, dadurch gekennzeichnet, daß $R^3$ einen freien oder geschützten Rest aus der Gruppe bestehend aus Ribose, Arabinose, Xylose, Lyxose, Allose, Altrose, Glucose, Mannose, Gulose, Idose, Galactose, Talose, Glucosamin, Galactosamin oder Mannosamin in der D-oder L-Form bedeutet.

4. Verbindungen nach den Ansprüchen 1 und 2, dadurch gekennzeichnet, daß $R^3$ einen Rest aus der Gruppe bestehend aus 1,2:3,4-Di-O-isopropyliden-$\alpha$-D-galactopyranose, 2,3:5,6-Di-O-isopropyliden-$\alpha$-D-mannofuranose, 1,2:5,6-Di-O-isopropyliden-$\alpha$-D-glucofuranose, 1,2,3,4-Tetra-O-acetyl-D-gluco-, -galacto-oder -mannopyranose, 2-Acetamido-1,3,4-tri-O-acetyl-2-desoxy-D-glucopyranose, 2,3,4,6-Tetra-O-benzyl-D-gluco-

, -galacto-oder -mannopyranose, 2,3,4,6-Tetra-O-acetyl-D-gluco-, -galacto-oder -mannopyranose, 2-Acetamido-3,4,6-tri-O-acetyl-2-desoxy-D-glucopyranose, Benzyl 3,4-O-isopropyliden-$\beta$-D-ribofuranosid bedeutet.

5. Verbindungen nach Anspruch 1, dadurch gekennzeichnet, daß

$R^1$ für Methyl, Ethyl, Propyl, Mehtylethyl, Butyl, Methylbutyl, Dimethylethyl, Pentyl, Methylbutyl, Ethylpropyl, Dimethylpropyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, Docosyl, Tetracosyl, Triacontyl, Ethylpentyl, Methldecyl, Propyldecyl, Methyltridecyl, Pentylhexadecyl, Docecylhexadecyl, Octadecyleicosyl, Hexadecyloctadecyl, Ethenyl, 1-Propenyl, 2-Propenyl, Butenyl, Mehtylpropenyl, Mehtylbutenyl, Pentenyl, Hexenyl, Octenyl, Nonenyl, Decenyl, 3,7-Dimethyl-2,6-octadienyl, Octadecenyl, Octadecadien-9,12-yl, Octadecantrien-9,12,15-yl, 3,7,11-Trimethyl-2,6,10-dodecatrien-1-yl, Butadienyl, Pentadienyl, Ethinyl, Propinyl, Butinyl, Pentinyl, Hexinyl, Heptinyl, Octinyl, Noninyl, Decinyl, Dodecinyl, Tetradecinyl, Hexadecinyl, Octadecinyl, Cyclopropyl, Cyclobutyl, Cyclopentyl, Cyclohexyl, Cycloheptyl, Cyclohexylcyclohexyl, Cyclopentylcycloheptyl, Methylcyclopentyl, Ethylcyclopentyl, n-Propylcyclopentyl, i-Propylcyclopentyl, Butylcyclopentyl, Octylcyclopentyl, Methylcyclohexyl, Ethylcyclohexyl, Propylcyclohexyl, Butylcyclohexyl, Hexylcyclohexyl, Decylcyclohexyl, Cyclopentylmethyl, Cyclopentylethyl, Cyclopentylpropyl, Cyclopentylbutyl, Cyclopentylpentyl, Cyclopentylhexyl, Cyclopentyloctyl, Cyclopentyldecyl, Cyclohexylmethyl, Cyclohexylethyl, Cyclohexylpropyl, Cyclohexylbutyl, Cyclohexyldecyl, Cyclopentylcyclohexylethyl, Cyclohexylcyclopentylethyl oder Cyclohexylcyclohexylethyl steht und

$R^2$ für Phenyl, o-Chlorphenyl, m-Chlorphenyl, p-Chlorphenyl, o-Fluorphenyl, m-Fluorphenyl, p-Fluorphenyl, o-Nitrophenyl, m-Nitrophenyl, p-Nitrophenyl, 2,4-Dichlorphenyl, Benzyl, p-Methoxybenzyl, 2,4-Dinitrophenyl, Benzhydryl, p-Methoxybenzyl, Triphenylmethyl, 2,2,2-Trichlorethyl, 2-Cyanoethyl, Wasserstoff, $Li^+$, $Na^+$, $K^+$, $Mg^{2+}$, $Ca^{2+}$, $NH_4^+$ Triethylammonium, Pyridinium, Tetraethylammonium, Tetrabutylammonium, Cyclohexylammonium oder Dicyclohexylammonium steht.

6. Verbindungen nach den Ansprüchen 1 bis 4, dadurch gekennzeichnet, daß $R^1$ vorzugsweise ein-oder zweifach durch Halogen, vorzugsweise F, Cl oder Br, Azido, Cyano oder Trialkylsilyl substituiert ist.

7. Verfahren zur Herstellung von Verbindungen der Formel (I) nach Anspruch 1, dadurch gekennzeichnet, daß man Zuckerkomponenten der Formel (V) mit einem Phosphorochloridat der Formel (IV) nach dem Schema (III) umsetzt

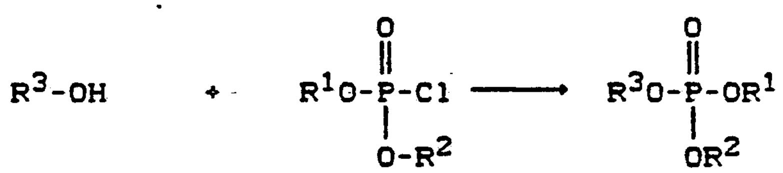

(III),

(V) (IV (I)

wobei $R^3$, $R^1$ und $R^2$ die in Anspruch 1 angegebene Bedeutung haben.

8. Verbindungen der allgemeinen Formel (I)

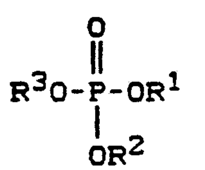

(I)

in welcher

$R^1$ Wasserstoff, einen gegebenenfalls substituierten geradkettigen oder verzweigten gesättigten oder einfach oder mehrfach ungesättigten Alkylrest, einen gegebenenfalls substituierten, gesättigten oder einen ein-oder mehrfach ungesättigten Cycloalkylrest,

$R^2$ Wasserstoff, Phosphatschutzgruppen oder ein anorganisches oder organisches Kation bedeutet und

$R^3$ einen freien oder geschützten Saccharidrest in der Pyranose-oder Furanoseform aus der Reihe der Hexosen oder Pentosen bedeutet,

zur Anwendung in einem Verfahren zur therapeutischen Behandlung des menschlichen oder tierischen Körpers.

9. Arzneimittel, enthaltend Verbindungen der allgemeinen Formel (I)

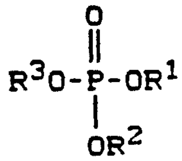

(I)

in welcher

$R^1$ Wasserstoff, einen gegebenenfalls substituierten geradkettigen oder verzweigten gesättigten oder einfach oder mehrfach ungesättigten Alkylrest, einen gegebenenfalls substituierten, gesättigten oder einen ein-oder mehrfach ungesättigten Cycloalkylrest,

$R^2$ Wasserstoff, Phosphatschutzgruppen oder ein anorganisches oder organisches Kation bedeutet und

$R^3$ einen freien oder geschützten Saccharidrest in der Pyranose-oder Furanoseform aus der Reihe der Hexosen oder Pentosen bedeutet.

10. Verwendung von Verbindungen der allgemeinen Formel (I)

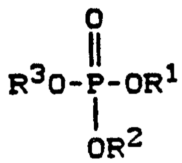

(I)

in welcher

$R^1$ Wasserstoff, einen gegebenenfalls substituierten geradkettigen oder verzweigten gesättigten oder einfach oder mehrfach ungesättigten Alkylrest, einen gegebenenfalls substituierten, gesättigten oder einen ein-oder mehrfach ungesättigten Cycloalkylrest,

$R^2$ Wasserstoff, Phosphatschutzgruppen oder ein anorganisches oder organisches Kation bedeutet und

$R^3$ einen freien oder geschützten Saccharidrest in der Pyranose-oder Furanoseform aus der Reihe der Hexosen oder Pentosen bedeutet,

zur Herstellung von Arznemitteln.